# EUROPEAN PATENT SPECIFICATION

(11) **EP 2 937 109 B1**
(45) Date of publication and mention of the grant of the patent: **24.07.2019**
(21) Application number: 15164613.0
(22) Date of filing: 22.04.2015
(51) Int. Cl.: A61M 25/09

(54) **GUIDEWIRE**
FÜHRUNGSDRAHT
FIL-GUIDE

(30) Priority: 24.04.2014 JP 2014089715
(43) Date of publication of application: 28.10.2015
(73) Proprietor: Asahi Intecc Co., Ltd., Seto-shi, Aichi 489-0071 (JP)
(72) Inventor: Murata, Satoru, Seto-shi, Aichi 489-0071 (JP); Koike, Tadahiro, Seto-shi, Aichi 489-0071 (JP); Ushida, Keisuke, Seto-shi, Aichi 489-0071 (JP)
(74) Representative: Winter, Brandl, Fürniss, Hübner, Röss, Kaiser, Polte - Partnerschaft mbB

(56) References cited:
- EP-A1- 0 410 602
- EP-A1- 2 005 988
- WO-A1-92/13483
- JP-B1- 5 448 125
- US-A1- 2011 060 316

## Description

### Field

The present invention relates to a guidewire for use as medical equipment that is inserted into body cavities for the purpose of treatment and examination.

### Background

Conventionally, all kinds of guidewires have been developed as a guide for a catheter or the like that is inserted into tubular organs such as blood vessels, the digestive tract, and the ureter, and into body tissue for the purpose of treatment and examination.

For example, Prior Art Document 1 discloses a guidewire that includes a core wire, an outer coil provided at a distal end portion of the core wire, and an inner coil provided within the outer coil.

### Citation List

### Patent Literature

Prior Art Document 1: Japanese Patent Application Publication No. 8-317989 (JP 8-317989 A)

### Summary

### Technical Problem

Usually in the guidewire disclosed by Prior Art Document 1, when torque operation is applied in such a direction that the outer coil becomes tightened, for example, single-strand wires of the outer coil are pressed to each other to increase contact pressure, which leads the outer coil to deform inwardly to reduce its diameter. When such deformation occurs to an excessive degree, a problem that a single-strand wire becomes displaced onto an adjacent single-strand wire can occur.

Incidentally, when inserted deep into a lesion that is firm, is narrowed to a great extent, or is obstructed, for example, a guidewire is required to exhibit adequate torque transmission. As a way to meet this requirement, a coil formed by winding a plurality of stranded wires, each formed of a plurality of strands twisted together, in a spiral manner is considered.

In such a coil, however, when torque operation is applied in such a direction that the coil becomes tightened, not only the strands are pressed to each other but also the stranded wires are pressed strongly to each other to increase contact pressure even further, which can lead the coil to deform inwardly to an excessive degree. This tends to cause a strand (or stranded wire) to become displaced onto an adjacent strand (or stranded wire), as described above. Thus, there is still room for improvement in solving such a problem that a strand (or stranded wire) becomes displaced onto an adjacent strand (or stranded wire).

The present invention was devised based on the above circumstances, and an object of the present invention is to provide a guidewire that can solve such a problem that a strand (or stranded wire) becomes displaced onto an adjacent strand (or stranded wire).

### Solution to Problem

In order to solve the above problem, a guidewire of the present invention has the following characteristics.

First aspect of the present invention refers to a guidewire including:
a shaft,
an outer coil wound around a distal end portion of the shaft, and
an inner coil wound around a distal end portion of the shaft, provided within the outer coil and being formed of at least one wire wound in a spiral manner,
the outer coil being formed of a plurality of stranded wires wound in a spiral manner, each of the stranded wires being formed of a plurality of strands twisted together,
the wire forming the inner coil being formed of a plurality of strands twisted together, and
a direction of winding of the outer coil being opposite to the direction of winding of the inner coil.

The at least one wire forming the inner coil may be a single-strand wire.

The inner coil may be formed of a plurality of wires.

### Advantageous Effects of Invention

The outer coil may be formed by winding a plurality of stranded wires, each formed of a plurality of strands twisted together, in a spiral manner, and the direction of winding of the outer coil is opposite to the direction of winding of the inner coil.

Usually, when torque operation is applied in such a direction that the outer coil becomes tightened, not only the strands are pressed to each other but also the stranded wires are pressed to each other to increase contact pressure, which leads the outer coil to deform inwardly to reduce its diameter. When such deformation occurs to an excessive degree, a problem that a strand (or stranded wire) becomes displaced onto an adjacent strand (or stranded wire) occurs.

However, the direction of winding of the outer coil is opposite to the direction of winding of the inner coil and therefore, when winding of the outer coil is tightened and the outer coil deforms inwardly to reduce its diameter, the inner coil is relaxed in terms of the close contact among its turns to increase its outer diameter. This leads both of the coils to interfere with each other to suppress excessive inward deformation of the outer coil. As a result, such a problem described above that a strand (stranded wire) becomes displaced onto an adjacent strand (stranded wire) can be avoided.

The inner coil of the guidewire may be formed of a plurality of wires. The wires are capable of slightly moving relative to each other. Therefore, when torque operation is applied in a particular direction, contact among the wires is readily relaxed to increase the outer diameter of the coil, which allows the range of diameter expansion to be greater.

In other words, when winding of the outer coil is tightened and the outer coil deforms inwardly to reduce its diameter, the outer diameter of the inner coil readily increases. This leads both of the coils to readily interfere with each other to effectively suppress excessive inward deformation of the outer coil. As a result, such a problem described above that a strand (or stranded wire) becomes displaced onto an adjacent strand (or stranded wire) can be reliably avoided.

According to the invention, the inner coil of the guidewire is formed by winding a plurality of stranded wires in a spiral manner. Thereby not only the stranded wires but also the strands that form the stranded wires are capable of slightly moving relative to each other, the coil has a degree of freedom and has improved flexibility. Therefore, when torque operation is applied in a particular direction, the inner coil is relaxed readily to easily increase its outer diameter, which allows the range of diameter expansion to be even greater.

In other words, when winding of the outer coil is tightened and the outer coil deforms inwardly to reduce its diameter, the outer diameter of the inner coil significantly increases. This results in both of the coils interfering with each other more reliably to suppress excessive inward deformation of the outer coil. As a result, such a problem described above that a strand (stranded wire) becomes displaced onto an adjacent strand (stranded wire) can be avoided even more reliably.

### Brief Description of Drawings

FIG. 1 is an expanded view of a partial cross-section of a guidewire of a first embodiment of the present invention.
FIG. 2 is a sectional view taken from line A-A of FIG. 1.
FIG. 3 is a cutaway side view of the guidewire of the first embodiment of the present invention.
FIG. 4 is an expanded view of a partial cross-section of a guidewire of a second embodiment of the present invention.
FIG. 5 is a perspective view of an inner coil of the second embodiment of the present invention.
FIG. 6 is a sectional view taken from line B-B of FIG. 4.
FIG. 7 is an expanded view of a partial cross-section of a guidewire of a third embodiment of the present invention.
FIG. 8 is a sectional view taken from line C-C of FIG. 7.

### Description of Embodiments

First, a guidewire of the present invention is explained referring to embodiments shown in the drawings.

### [First embodiment]

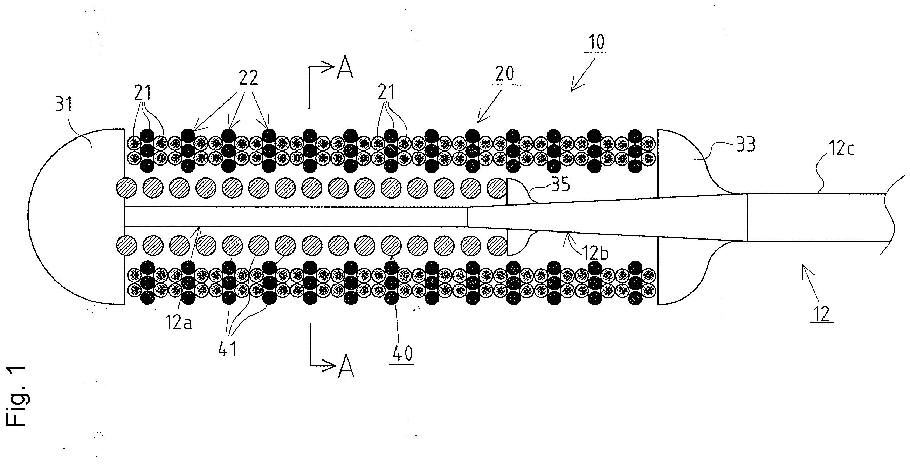

FIG. 1 is an expanded view of a partial cross-section of a first embodiment of a guidewire of the present invention. In FIG. 1, the distal end side to be inserted into the body is shown on the left hand side, and the proximal end side to be handled by an operator such as a doctor is shown on the right hand side. This drawing is merely a schematic view of the guidewire and the dimensional ratios including the cross-sectional profile of a coil formed of stranded wires are different from the actual dimensional ratios.

A guidewire 10 shown in FIG. 1 is used, for example, in treating blood vessels of a lower limb with the Cross Over technique. The guidewire 10 includes a shaft 12 and an outer coil 20 covering the outer circumference of a distal end portion of the shaft 12.

The shaft 12 includes a thin portion 12a, a tapered portion 12b, and a greater-diameter portion 12c in this order from the distal end to the proximal end side. The thin portion 12a is a portion of the shaft 12 at the most distal end side and is the most flexible part of the shaft 12. The thin portion 12a is formed flat by pressing. The tapered portion 12b is tapered with a circular cross section, with its diameter reduced toward the distal end side. The greater-diameter portion 12c has a diameter greater than the diameter of the thin portion 12a.

The material to form the shaft 12 is not particularly limited and can be a stainless steel (SUS304), a super-elastic alloy such as Ni-Ti alloys, piano wire, a cobalt-based alloy, or the like.

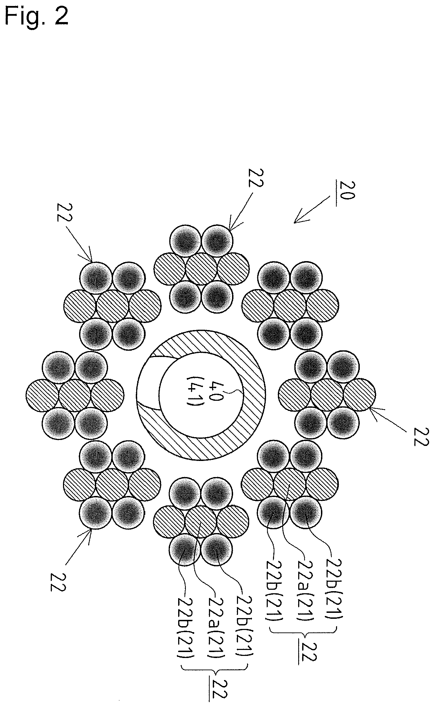

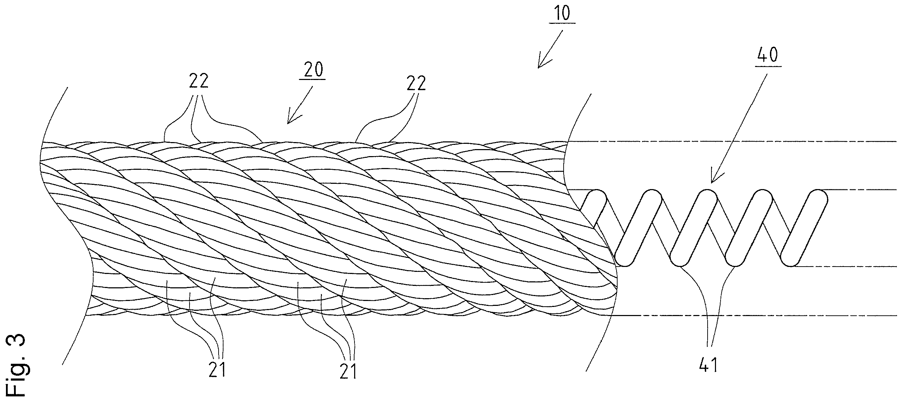

As shown in FIG. 1 and FIG. 2, the outer coil 20 of this embodiment is formed by winding a plurality of stranded wires 22 (eight stranded wires 22 in this embodiment), each formed of a plurality of strands 21 twisted together, in a spiral manner. As shown in FIG. 2, each of the stranded wires 22 includes a core strand 22a and six peripheral strands 22b covering the outer circumference of the core strand 22a. In this embodiment, as shown in FIG. 3, the direction of winding of the outer coil 20 is a clockwise direction to the right in the drawing (Define the direction as Z direction).

The materials to form the core strand 22a and the peripheral strands 22b are not particularly limited and examples thereof include stainless steels such as martensitic stainless steel, ferritic stainless steel, austenitic stainless steel, austenitic-ferritic duplex stainless steel, and precipitation hardened stainless steel, super-elastic alloys such as Ni-Ti alloys, and metals radiopaque to X-rays such as platinum, gold, tungsten, tantalum, and iridium, and alloys thereof.

The distal end of the outer coil 20 is fixed to the distal end of the shaft 12 via a distal end bonding member 31. The proximal end of the outer coil 20 is fixed to the shaft 12 via a proximal end bonding member 33. The materials to form the distal end bonding member 31 and the proximal end bonding member 33 are not particularly limited and examples thereof include brazing metals such as Sn-Pb alloys, Pb-Ag alloys, Sn-Ag alloys, and Au-Sn alloys.

The guidewire 10 of this embodiment includes an inner coil 40 within the outer coil 20. The inner coil 40 of this embodiment is a single-strand coil formed by winding a wire 41 in a spiral manner. The wire 41 is formed of a single strand. I.e., the inner coil 40 is formed of one single-strand wire.

The inner coil 40 is not particularly limited in terms of its material and can be formed with a radiopaque wire or a radiolucent wire. The material to form the radiopaque wire is not particularly limited and can be gold, platinum, tungsten, an alloy containing such an element (a platinum-nickel alloy, for example), or the like. The material to form the radiolucent wire is not particularly limited and can be a stainless steel (SUS304 or SUS316, for example), a super-elastic alloy such as Ni-Ti alloys, piano wire, or the like.

The distal end of the inner coil 40 is bonded to the distal end of the shaft 12 via the distal end bonding member 31. The proximal end of the inner coil 40 is bonded to the shaft 12 via a proximal end bonding member 35. The material to form the proximal end bonding member 35 is not particularly limited and examples thereof include brazing metals such as Sn-Pb alloys, Pb-Ag alloys, Sn-Ag alloys, and Au-Sn alloys.

As shown in FIG. 3, the direction of winding of the inner coil 40 is a counterclockwise direction to the right in the drawing (Define the direction as S direction). In other words, the direction of winding of the outer coil 20 is opposite to the direction of winding of the inner coil 40.

Usually, when torque operation is applied in such a direction that the outer coil 20 becomes tightened, not only the strands 21 are pressed to each other but also the stranded wires 22 are pressed to each other to increase contact pressure, which leads the outer coil 20 to deform inwardly to reduce its diameter. When such deformation occurs to an excessive degree, a problem that a strand 21 (or stranded wire 22) becomes displaced onto an adjacent strand 21 (or stranded wire 22) can occur.

In the present invention, however, the direction of winding of the outer coil 20 is opposite to the direction of winding of the inner coil 40 and therefore, when winding of the outer coil 20 is tightened and the outer coil 20 deforms inwardly to reduce its diameter, the inner coil 40 is relaxed in terms of the close contact among its turns to increase its outer diameter. This leads both of the coils 20 and 40 to interfere with each other to suppress excessive inward deformation of the outer coil 20. As a result, such a problem described above that a strand 21 (or stranded wire 22) becomes displaced onto an adjacent strand 21 (or stranded wire 22) can be avoided.

### [Second embodiment]

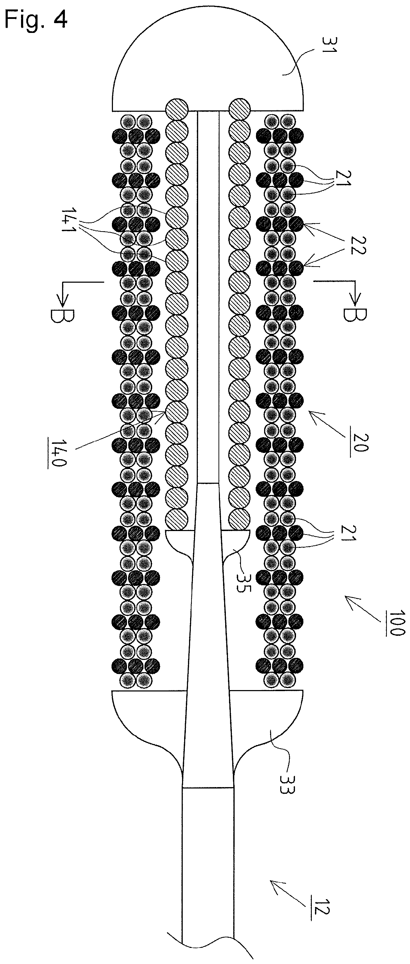

FIG. 4 is an expanded view of a partial cross-section of a guidewire of a second embodiment of the present invention. In FIG. 4, the distal end side to be inserted into the body is shown on the left hand side, and the proximal end side to be handled by an operator such as a doctor is shown on the right hand side. This drawing is merely a schematic view of the guidewire and the dimensional ratios including the cross-sectional profiles of the coils formed of a stranded wire or stranded wires are different from the actual dimensional ratios.

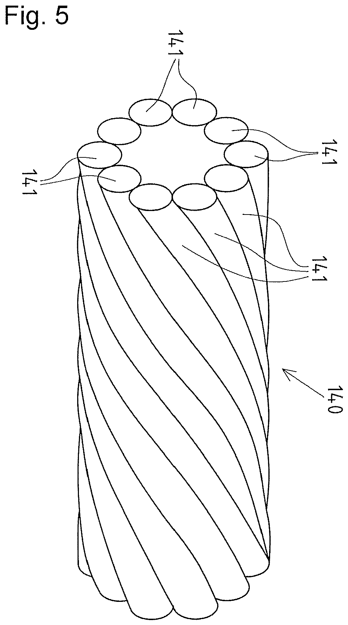

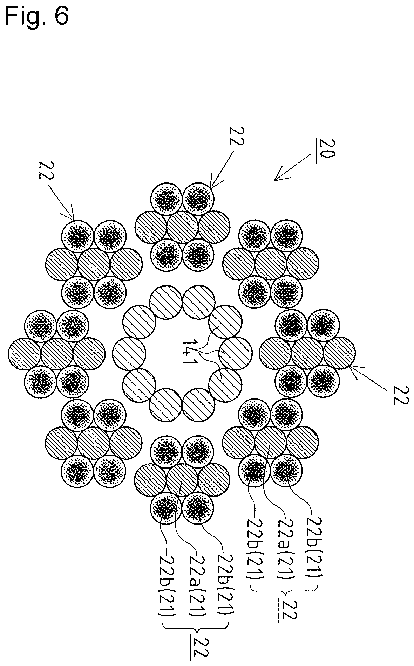

In the first embodiment, the inner coil 40 used is a single-strand inner coil formed by winding one single-strand wire in a spiral manner. A guidewire 100 of this embodiment, however, as shown in FIG. 5 and FIG. 6, includes a hollow inner coil 140 that is formed of a plurality of single-strand wires 141 (ten single-strand wires 141 in this embodiment) twisted together.

The wires 141 are capable of slightly moving relative to each other. Therefore, when torque operation is applied in a particular direction, contact among the wires 141 is readily relaxed to increase the outer diameter of the coil, which allows the range of diameter expansion to be greater.

In other words, when winding of the outer coil 20 is tightened and the outer coil 20 deforms inwardly to reduce its diameter, the outer diameter of the inner coil 140 readily increases compared to the outer diameter of the inner coil of the first embodiment. This leads both of the coils 20 and 140 to readily interfere with each other to effectively suppress excessive inward deformation of the outer coil 20. As a result, such a problem described above that a strand 21 (or stranded wire 22) becomes displaced onto an adjacent strand 21 (or stranded wire 22) can be reliably avoided.

### [Third embodiment]

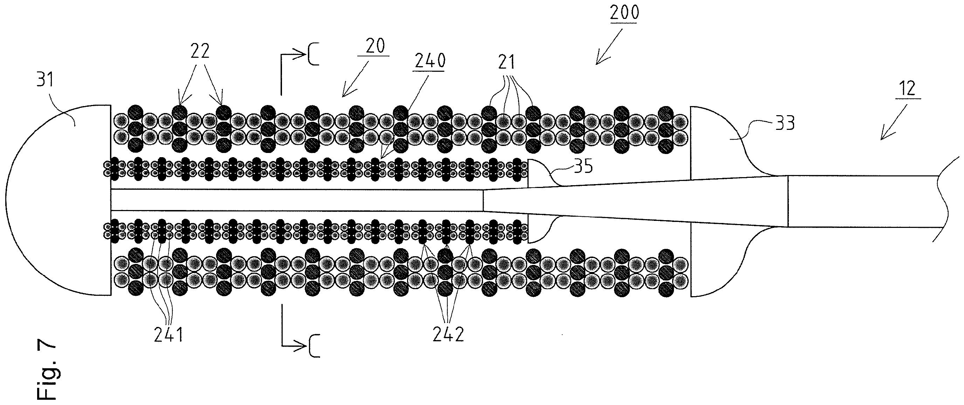

FIG. 7 is an expanded view of a partial cross-section of a guidewire of a third embodiment of the present invention. In FIG. 7, the distal end side to be inserted into the body is shown on the left hand side, and the proximal end side to be handled by an operator such as a doctor is shown on the right hand side. This drawing is merely a schematic view of the guidewire and the dimensional ratios including the cross-sectional profiles of the coils formed of stranded wires are different from the actual dimensional ratios.

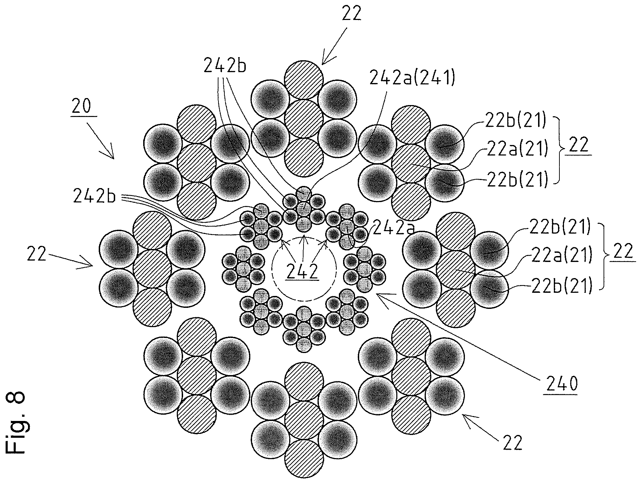

As shown in FIG. 7 and FIG. 8, an inner coil 240 of a guidewire 200 of this embodiment is formed by winding a plurality of stranded wires 242 (eight stranded wires 242 in this embodiment), each formed of a plurality of strands 241 twisted together, in a spiral manner. More specifically, as shown in FIG. 8, the inner coil 240 is formed by winding eight stranded wires 242, each formed of a core strand 242a wound together with six peripheral strands 242b that cover the outer circumference of the core strand 242a, in a spiral manner.

The materials to form the core strand 242a and the peripheral strands 242b in the inner coil 240 are not particularly limited and examples thereof include stainless steels such as martensitic stainless steel, ferritic stainless steel, austenitic stainless steel, austenitic-ferritic duplex stainless steel, and precipitation hardened stainless steel, super-elastic alloys such as Ni-Ti alloys, and metals radiopaque to X-rays such as platinum, gold, tungsten, tantalum, and iridium and alloys thereof.

Not only the stranded wires 242 but also the strands 241 that form the stranded wires 242 are capable of slightly moving relative to each other. Thus, the inner coil 240 with such a configuration has a degree of freedom and is further improved in flexibility compared to the inner coil of the second embodiment. Therefore, when torque operation is applied in a particular direction, the inner coil 240 is relaxed even more readily to easily increase its outer diameter, which allows the range of diameter expansion to be even greater.

In other words, when winding of the outer coil 20 is tightened and the outer coil 20 deforms inwardly to reduce its diameter, the outer diameter of the inner coil 240 significantly increases compared to the outer diameter of the inner coil of the second embodiment. This results in both of the coils 20 and 240 interfering with each other more reliably to suppress excessive inward deformation of the outer coil 20. As a result, such a problem described above that a strand 21 (stranded wire 22) becomes displaced onto an adjacent strand 21 (stranded wire 22) can be avoided even more reliably.

### Reference Signs List

10, 100, 200: Guidewire
12: Shaft
20: Outer coil
21: Strand to form outer coil
22: Stranded wire to form outer coil
40, 140, 240: Inner coil
41, 141: Wire to form inner coil
241: Strand to form stranded wire
242: Stranded wire to form inner coil

## Claims

1. A guidewire (10, 100, 200) comprising:
a shaft (12),
an outer coil (20) wound around a distal end portion of the shaft (12), and
an inner coil (40, 140, 240) wound around a distal end portion of the shaft (12), provided within the outer coil (20) and being formed of at least one wire (41, 141, 242) wound in a spiral manner, **characterized by**
the outer coil (20) being formed of a plurality of stranded wires (22) wound in a spiral manner, each of the stranded wires (22) being formed of a plurality of strands (22a, 22b) twisted together,
the wire (242) forming the inner coil (240) being formed of a plurality of strands (242a, 242b) twisted together, and
a direction of winding of the outer coil (20) being opposite to a direction of winding of the inner coil (40, 140, 240).

2. The guidewire (10) according to claim 1, wherein the inner coil (40) is formed of one wire (41).

3. The guidewire (100, 200) according to claim 1, wherein the inner coil (140, 240) is formed of a plurality of wires (141, 242).

## Patentansprüche

1. Führungsdraht (10, 100, 200) mit:
einem Schaft (12),
einer äußeren Wicklung (20), die um einen distalen Endabschnitt des Schafts (12)gewickelt ist, und
einer inneren Wicklung (40, 140, 240), die um den distalen Endabschnitt des Schafts (12) gewickelt, innerhalb der äußeren Wicklung (20) angeordnet und aus wenigstens einem schraubenförmig gewickelten Draht (41, 141, 242) gebildet ist, **dadurch gekennzeichnet, dass**
die äußere Wicklung (20) aus einer Vielzahl schraubenförmig gewickelter Litzendrähte (22) gebildet ist, die jeweils aus einer Vielzahl von miteinander verdrillten Litzen (22a, 22b) gebildet sind,
der Draht (242), der die innere Wicklung (240) bildet, aus einer Vielzahl miteinander verdrillter Litzen (242a, 242b) gebildet ist, und
die Wicklungsrichtung der äußeren Wicklung (20) gegensinnig zur Wicklungsrichtung der inneren Wicklung (40, 140, 240) ist.

2. Führungsdraht (10) nach Anspruch 1, wobei die innere Wicklung (40) aus genau einem Draht (41) gebildet ist.

3. Führungsdraht (100, 200) nach Anspruch 1, wobei die innere Wicklung (140, 240) aus einer Vielzahl von Drähten (141, 242) gebildet ist.

## Revendications

1. Fil de guidage (10, 100, 200) comprenant :
un arbre (12),
une bobine extérieure (20) enroulée autour d'une partie d'extrémité distale de l'arbre (12), et
une bobine intérieure (40, 140, 240) enroulée autour d'une partie d'extrémité distale de l'arbre (12), disposée dans la bobine extérieure (20) et étant formée d'au moins un fil (41, 141, 242) enroulé d'une manière en spirale, **caractérisé en ce que**
la bobine extérieure (20) est formée d'une pluralité de fils toronnés (22) enroulés d'une manière en spirale, chacun des fils toronnés (22) étant formé d'une pluralité de torons (22a, 22b) torsadées ensemble,
le fil (242) formant la bobine intérieure (240) étant formé d'une pluralité de torons (242a, 242b) torsadés ensemble, et
une direction d'enroulement de la bobine extérieure (20) étant opposée à une direction d'enroulement de la bobine intérieure (40, 140, 240).

2. Fil de guidage (10) selon la revendication 1, dans lequel la bobine intérieure (40) est formée d'un fil (41).

3. Fil de guidage (100, 200) selon la revendication 1, dans lequel la bobine intérieure (140, 240) est formée d'une pluralité de fils (141, 242).
